# FASCICULE DE BREVET EUROPEEN

(11) **EP 3 016 933 B1**
(45) Date de publication et mention de la délivrance du brevet: **26.12.2018**
(21) Numéro de dépôt: 14736745.2
(22) Date de dépôt: 03.07.2014
(51) Int. Cl.: C07C 309/80, C07C 309/81, C07C 303/02, C07C 303/38, C07C 309/06, C07C 311/48

(54) **PROCÉDÉ NON-ÉLECTROCHIMIQUE DE FLUORATION DE COMPOSÉS HALOGÉNURES DE SULFONYLE**
NICHT-ELETROCHEMISCHES VERFAHREN ZUR FLUORIERUNG VON SULFONYL-HALOGENID-VERBINDUNGEN
NON-ELECTROCHEMICAL PROCESS FOR FLUORINATION OF SULPHONYL HALIDE COMPOUNDS

(30) Priorité: 04.07.2013 FR 1301593
(43) Date de publication de la demande: 11.05.2016
(73) Titulaire: Rhodia Operations, 75009 Paris (FR)
(72) Inventeur: METZ, François, F-69540 Irigny (FR)
(74) Mandataire: Menville, Laure
(86) Numéro de dépôt international: PCT/EP2014/064183
(87) Numéro de publication internationale: WO 2015/001020

(56) Documents cités:
- DE-C- 907 775
- DE-C- 936 090
- US-A- 2 276 097
- US-A- 5 723 664
- KAGEYAMA H ET AL: "Sulfonyl chloride as a disposable electron withdrawing substituent in halex fluorinations", JOURNAL OF FLUORINE CHEMISTRY, ELSEVIER, NL, vol. 101, no. 1, 1 janvier 2000 (2000-01-01), pages 85-89, XP004244500, ISSN: 0022-1139, DOI: 10.1016/S0022-1139(99)00195-5
- IL'CHENKO A Y ED - BELLUS D (ED): "Tetrahetero-substituted methanes with a carbon-halogen bond", 1 janvier 2005 (2005-01-01), SCIENCE OF SYNTHESIS. CATEGORY 3 : COMPOUNDS WITH FOUR AND THREE CARBON-HETEROATOM BONDS; [METHODS OF MOLECULAR TRANSFORMATIONS. (HOUBEN-WEYL)], STUTTGART : GEORG THIEME VERLAG, DE, PAGE(S) 1135 - 1201, XP008106589, ISBN: 978-3-13-118681-2 page 1169 - page 1170

## Description

La présente invention relève du domaine de la fluoration de composés halogénés, généralement de composés chlorés, portant une fonction -SO₂X où X est un halogène différent du fluor. L'invention a notamment pour objet la préparation de composés de type fluorure de sulfonyle tels que les fluorures d'alkylsulfonyle ou les fluorures de benzylsulfonyle. Plus précisément, la présente invention a pour objet un procédé de préparation du fluorure de trifluorométhane sulfonyle (CF₃SO₂F).

Les composés fluorures de sulfonyle sont des intermédiaires particulièrement intéressants pour la synthèse de composés sulfonimides et de composés portant une fonction acide sulfonique qui sont des produits à haute valeur ajoutée.

Il est connu de préparer les composés de type fluorure de sulfonyle et en particulier le fluorure de trifluorométhane sulfonyle par électrofluoration du fluorure de mésyle CH₃SO₂F (US 4.927.962) ou par fluoruration d'un trifluorosulfinate d'un cation monovalent. Le brevet DE 936 090 décrit qu'un halogénure de β,β,β-trichloro-éthane sulfonyle pouvait subir une réaction d'échange chlore/fluor par réaction avec du HF en présence de pentachlorure d'antimoine. Le brevet US 2,276,097 décrit quant à lui la préparation de fluorures de sulfonyle chlorés à partir de chlorures de sulfonyle chlorés. En outre, la publication scientifique de A. Y. II'chenko (« Tetrahetero-substituted methanes with a carbon-halogen bond», 1 January 2005, in SCIENCE OF SYNTHESIS, HOUBEN-WEYL 18, Compounds with Four and Three Cabon-Heteroatom Bonds, pages 1135 - 1201) fait un état des lieux des connaissances sur les composés tétrahalogénure de carbone. Ces procédés antérieurs, même s'ils conduisent à des performances satisfaisantes, souffrent d'une mise en oeuvre complexe et onéreuse (cas de l'électrofluoration) et/ou nécessitant la mise en place d'étapes faisant intervenir des solvants CMR (cancérigène, mutagène et reprotoxique), en particulier le DMF.

Un objectif de l'invention est donc de fournir un procédé de préparation de composés fluorures de sulfonyle qui permet de s'affranchir des inconvénients des procédés de l'état de la technique. En particulier, un objectif de l'invention est de proposer un procédé simplifié de préparation de composés de type fluorure de sulfonyle, plus propre en termes de toxicité des agents chimiques impliqués et plus économique. Le procédé de l'invention est un procédé non-électrochimique. D'autres objectifs encore apparaîtront à la lecture de l'invention qui suit.

La présente invention a pour objet un procédé non-électrochimique de préparation d'un composé fluoré de formule (I) comportant au moins une fonction -SO₂F caractérisé en ce que le composé de formule (I) est préparé par réaction d'un composé de formule (II) avec au moins un agent de fluoration choisi parmi l'acide fluorhydrique et un fluorure ionique d'un cation monovalent ou divalent :

R-SO₂F (I)

où R est choisi parmi les groupements R1, R2 et R3 suivants :
R1 = -CₙHₐF_{b} avec n=1, a+b = 3, b ≥ 1 ; De préférence : n=1 , a = 0, b = 3
R2 = -CₓH_{y}F_{z}-SO₂F avec x = 1, y+z = 2 et z ≥ 1 ;
R3 = Φ-C_{c}HₕF_{f} avec c = 1 ; h+f = 2 et f ≥ 1 ; Φ désignant un groupe phényle ;

R'-SO₂X (II)

où R' est choisi parmi les groupements R'1, R'2 et R'3 suivants :
R'1 = -CₙHₐX_{b} avec n=1, a+b = 3, b ≥ 1 ;
R'2 = -CₓH_{y}X_{z}-SO₂X avec x = 1, y+z = 2 et z ≥ 1 ;
R'3 = Φ-C_{c}HₕX_{f} avec c = 1 ; h+f = 2 et f ≥ 1 ; Φ désignant un groupe phényle,
X étant un atome d'halogène choisi parmi le chlore et le brome.

Le procédé de préparation selon l'invention est un procédé de fluoration non-électrochimique. Les procédés d'électrofluoration sont donc exclus de l'invention.

De manière préférée, les radicaux R1 et R'1 sont perhalogénés de sorte que b = 3 et a = 0. Le groupe phényle φ présent dans les radicaux R3 et R'3 peut être substitué par un ou plusieurs groupes hydroxyles, alkyles, alcools, thiols, amides, halogènes et/ou par un groupement -CₓH_{y}X_{z}-SO₂X avec x = 1-10, y+z = 2x et z ≥ 1, X étant un atome d'halogène choisi parmi le chlore et le brome.

De manière très préférée, le radical R du composé (I) préparé selon le procédé de l'invention est le radical R1 dans lequel n = 1, a = 0 et b = 3, ou n = 1, a = 1, b = 2 ou encore n = 1, a = 2 et b = 1. On prépare ainsi respectivement les composés de formule CF₃SO₂F, CHF₂SO₂F et CH₂FSO₂F à partir des composés de formule (II) ayant respectivement pour formule CX₃SO₂X (n = 1, a = 0 et b = 3 dans R'1), CHX₂SO₂X (n = 1, a = 1, b = 2 dans R'1) et CH₂XSO₂X (n = 1, a = 2 et b = 1 dans R'1), où X est le brome ou le chlore, préférentiellement le chlore.

Par ailleurs, on décrit un procédé non-électrochimique de préparation d'un composé fluoré de formule SO₂F₂ caractérisé en ce que ce composé est préparé par réaction d'un composé de formule SO₂X₂ avec au moins un agent de fluoration choisi parmi l'acide fluorhydrique et un fluorure ionique d'un cation monovalent ou divalent, X étant un atome d'halogène choisi parmi le chlore et le brome.

Le procédé selon l'invention peut être réalisé en phase gazeuse ou en phase liquide. De manière préférée, ledit procédé est réalisé en phase gazeuse.

Selon le mode préféré de réalisation du procédé de l'invention selon lequel il est opéré en phase gazeuse, l'agent de fluoration employé pour réagir avec le composé de formule (II) est l'acide fluorhydrique.

Le procédé de préparation selon l'invention, mis en oeuvre en phase gazeuse en présence d'acide fluorhydrique, met en oeuvre au moins un catalyseur de fluoration. Ledit catalyseur de fluoration, mis en oeuvre dans le procédé de fluoration en phase gazeuse, est notamment choisi parmi les catalyseurs comprenant, ou consistant en, du chrome, du zinc, du nickel, un mélange de chrome et de zinc ou un mélange de chrome et de nickel.
Le catalyseur de fluoration peut être notamment un catalyseur à base de chrome. Le catalyseur mis en oeuvre est un oxyde de chrome massique (c'est-à-dire un catalyseur ne comprenant que l'élément métallique et de l'oxygène) ou comprend de préférence des oxydes, des halogénures, des oxyhalogénures ou sels minéraux de chrome, éventuellement dopé par un élément métallique tel que par exemple le nickel, le cobalt, le magnésium et le zinc). Il s'agit préférentiellement d'un oxyde de chrome, d'un fluorure de chrome ou d'un oxyfluorure de chrome qui peut éventuellement être dopé par un élément métallique par exemple le nickel ou le zinc.
Le catalyseur de fluoration peut subir une activation par un traitement thermique. En particulier, l'activation peut avoir lieu lors du procédé de fluoration. La température est avantageusement choisie entre 100 et 400°C, de préférence entre 200 et 300°C.
On utilise en particulier le chrome sous forme d'oxydes sous différents degrés d'oxydation et/ou sous forme d'hydroxydes sous forme de poudre ou de gel.
Il est possible de mettre en oeuvre un oxyde de chrome (III) activé, préparé par exemple par précipitation de sels de chrome (III) hydrosolubles, comme par exemple les chlorures, nitrates, acétates, sulfates à l'aide d'une solution aqueuse d'hydroxyde d'ammonium ou à l'aide d'une solution aqueuse d'un hydroxyde de métal alcalin, de préférence le sodium ou le potassium. Le précipité est séché à environ 110°C et calciné à une température inférieure à 700°C, de préférence comprise entre 200 et 600°C, On entend par chrome (III) le chrome à l'état d'oxydation (III).
L'oxyde de chrome anhydre peut être obtenu par calcination de sels de chrome inorganiques comme le chromate d'ammonium ou le nitrate de chrome ou par calcination de sels de chrome organiques comme par exemple, les oxalates ou formiates de chrome à une température comprise entre 200 et 500°C, de préférence entre 250 et 450°C, et de manière encore plus préférée entre 250 et 400°C, sous atmosphère d'azote.
Le catalyseur de fluoration peut également être un catalyseur de type Cr-Ni, avec une valence du chrome comprise entre 2 et 6 et celle du nickel comprise entre 0 et 2, la quantité de nickel exprimé en pourcentage atomique représentant de 0,1 à 50%. Un mode de préparation de ce catalyseur consiste à faire une décomposition thermique, séparément ou en mélange d'un ou plusieurs sels organiques du chrome (par exemple oxalate) et d'un sel ou plusieurs sels du nickel (par exemple oxalate), mise en forme du mélange. La décomposition thermique a lieu généralement entre 200 et 600°C, sous atmosphère de gaz inerte, par exemple l'azote.
La mise en forme du catalyseur obtenu peut être faite, dans des conditions non oxydantes, par exemple par extrusion puis le produit mis en forme est séché vers 80-150°C, puis calciné à 200-600°C, sous atmosphère inerte.
Un catalyseur de type Cr-Mg peut également être mis en oeuvre. Il peut être obtenu notamment par un mélange d'un sel de chrome (par exemple nitrate) en solution avec un oxyde ou hydroxyde de magnésium, séchage prolongé ente 12 et 24 heures par exemple à 100°C.
Le catalyseur de fluoration peut également être un catalyseur à base de chrome et de zinc. Le catalyseur mis en oeuvre est un zinc massique ou comprend de préférence des oxydes, des halogénures, des oxyhalogénures ou sels minéraux de zinc, éventuellement dopé par un élément métallique tel que par exemple le nickel, le cobalt ou le magnésium.
Le catalyseur de fluoration peut également être un catalyseur à base de nickel. Le catalyseur mis en oeuvre est un nickel massique ou comprend de préférence des oxydes, des halogénures, des oxyhalogénures ou sels minéraux de nickel, éventuellement dopé par un élément métallique tel que par exemple le zinc, le cobalt ou le magnésium.
Dans le catalyseur de fluoration de l'invention, la phase active peut être apportée sous une forme finement divisée ou bien mise en forme ou déposée sur un support. Comme exemples de supports, on peut mentionner la silice, l'alumine, l'alumine partiellement ou totalement fluorée, la zircone, l'oxyde de titane. De préférence, le catalyseur est déposé sur un support à raison de 0,2 à 15% du poids du catalyseur. Les catalyseurs supportés sont préparés selon des procédés bien connus de l'homme du métier et notamment par imprégnation ou co-imprégnation à humidité naissante sur le support de précurseurs métalliques dissouts dans un volume adapté d'eau.
Les catalyseurs peuvent se présenter sous différentes formes dans le procédé de l'invention : poudre, produits mis en forme tels que granulés (par exemple extrudés ou billes), pastilles, qui sont obtenus par extrusion, pastillage, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

Conformément au mode de réalisation mis en oeuvre en phase gazeuse en présence d'acide fluorhydrique, le rapport entre l'acide fluorhydrique et le composé de formule (II) peut varier largement. Généralement, la quantité de l'acide fluorhydrique est excédentaire. Ainsi, le rapport entre le nombre de moles d'acide fluorhydrique et le nombre de moles de composé halogéné de formule (II) varie le plus souvent entre 1 et 30. Il est avantageusement choisi entre 6 et 12.
Le procédé de l'invention, mis en oeuvre en phase gazeuse, est réalisé à une température élevée, en règle générale supérieure à 50°C. Il est recommandé de travailler à des températures comprises entre 50°C et 400°C, de préférence comprise entre 100°C et 300°C. Pour des raisons de simplicité, on réalise le procédé de l'invention sous la pression atmosphérique. Toutefois, il est également possible d'opérer sous des pressions plus basses ou plus élevées.
D'un point de vue pratique, le procédé de l'invention, mis en oeuvre en phase gazeuse, peut être mis en oeuvre en discontinu ou en continu.
De manière générale, on commence par effectuer le mélange d'une façon quelconque, du composé halogéné de formule (II) et de l'acide fluorhydrique. Ainsi, on peut mélanger lesdits réactifs, dans une zone de mélange, puis envoyer le mélange obtenu sur le lit catalytique. Lorsque l'on réalise le procédé en discontinu, la quantité de catalyseur de fluoration mis en oeuvre, exprimée en poids de catalyseur par poids du composé halogéné de formule (II) peut varier, par exemple, entre 0,1 et 20 %, de préférence entre 0,5 et 5 %.
L'autre variante de l'invention consiste à conduire la réaction en continu, dans un réacteur tubulaire ou multitubulaire comportant le catalyseur solide disposé en lit fixe. La quantité de catalyseur de fluoration mis en oeuvre, exprimée en poids de catalyseur par poids du composé halogéné de formule (II) est préférentiellement inférieure à 0,1 % poids.
Le composé halogéné de formule (II) et l'acide fluorhydrique peuvent être introduits séparément ou en mélange dans le réacteur. Comme mentionné précédemment, on peut les mélanger dans une zone de mélange, puis envoyer le mélange obtenu sur le lit catalytique. Le mélange réactionnel traverse le lit catalytique, de préférence, de bas en haut.
Le temps de contact qui est défini comme le rapport entre le volume apparent de catalyseur et le débit du flux gazeux peut varier largement, et est le plus souvent compris entre 0,2 et 100 secondes. Le temps de contact est choisi de préférence entre 5 et 50 secondes.

Le poids de substrat mis en oeuvre par poids de catalyseur et par heure varie généralement entre 0,01 h⁻¹ et 2 h⁻¹, de préférence entre 0,05 h⁻¹ et 0,5 h⁻¹.

En fin de réaction, on récupère une phase gazeuse comprenant l'acide fluorhydrique en excès, l'acide chlorhydrique formé par la réaction, et éventuellement le composé fluoré de formule (I) en fonction de son point d'ébullition. Ledit composé de formule (I), s'il présente un point d'ébullition élevé, se retrouve en phase liquide par condensation. De manière préférée, ledit composé de formule (I) est présent dans la phase gazeuse, en particulier lorsqu'il s'agit du fluorure de trifluorométhane sulfonyle.
Le composé de formule (I) est récupéré à partir du mélange réactionnel selon toutes les techniques classiques connues de l'Homme du métier. Par exemple, le flux gazeux comprenant le composé de formule (I) est mis en contact avec de l'eau dans laquelle HF et HCl sont absorbés. Ledit composé de formule (I) est facilement et préférentiellement récupéré sous forme liquide par condensation.

Selon un autre mode préféré de réalisation du procédé de l'invention selon lequel il est opéré en phase liquide, l'agent de fluoration employé pour réagir avec le composé de formule (II) est l'acide fluorhydrique ou au moins un fluorure ionique d'un cation monovalent ou divalent.

Conformément au mode de réalisation selon lequel le procédé de l'invention est mis en oeuvre en phase liquide en présence d'acide fluorhydrique, ledit procédé est opéré au moyen d'un catalyseur de fluoration à base d'antimoine. En particulier, ledit catalyseur est choisi parmi les fluorures d'antimoine SbF₃, SbF₄Cl et SbF₅, seul ou en mélange. De manière préférée, ledit catalyseur consiste essentiellement en l'espèce SbF₅ ou est un mélange des espèces SbF₃ et SbF₅. Ledit catalyseur peut être massique ou supporté sur un support tel que le noir de carbone, le graphite, l'alumine ou une alumine fluorée. La quantité de catalyseur de fluoration mis en oeuvre, exprimée en poids de catalyseur par poids du composé halogéné de formule (II) est préférentiellement inférieure comprise entre 0,01 et 10% poids, de préférence entre 0,1 et 5% poids.
Le procédé de fluoration selon l'invention, mis en oeuvre en phase liquide, peut être mis en oeuvre en discontinu ou en continu. Il est mis en oeuvre en présence de un ou plusieurs solvants, en particulier un excès d'acide fluorhydrique.
La mise en oeuvre en phase liquide du procédé de préparation selon l'invention, en présence d'acide fluorhydrique, est opérée à une température comprise entre 0 et 300°C, de préférence entre 50 et 150 °C. Cette mise en oeuvre est opérée sous pression autogène. Le rapport entre le nombre de moles d'acide fluorhydrique et le nombre de moles de composé halogéné de formule (II) varie le plus souvent entre 1 et 20. II est avantageusement choisi entre 3 et 10. Conformément au mode de réalisation selon lequel le procédé de l'invention est mis en oeuvre en phase liquide en présence d'un fluorure ionique d'un cation monovalent ou divalent, ledit fluorure ionique monovalent peut être un fluorure alcalin ou un fluorure d'un cation onium et ledit fluorure ionique d'un cation divalent est préférentiellement un fluorure alcalino-terreux ou un fluorure d'un cation appartenant au groupe IIB de la classification périodique des éléments. En soumettant le composé de formule (II) à une réaction d'échange entre le(s)dit(s) atome(s) d'halogène X présent dans ledit composé de formule (II) (X différent du fluor) et le fluor introduit par le fluorure ionique, le composé de formule (I) est préparé.

De manière préférée, ledit fluorure ionique d'un cation monovalent est tel que ledit cation monovalent est un cation alcalin choisi parmi le lithium, le sodium, le potassium et le césium. Très préférentiellement, il s'agit du potassium. Ledit fluorure ionique d'un cation monovalent peut encore être un fluorure d'un cation onium, c'est-à-dire un fluorure d'ammonium dans lequel le cation répond à la formule N(R₄R₅R₆R₇)⁺ ou un fluorure de phosphonium dans lequel le cation répond à la formule P(R₄R₆R₆R₇)⁺, R₄, R₅, R₆ et R₇, identiques ou différents, sont choisis parmi un groupe alkyle, linéaire ou ramifié, ayant 1 à 12 atomes de carbone, de préférence de 1 à 4 atomes de carbone, et un groupe benzyle.
A titre d'exemples plus spécifiques, on peut citer les fluorures de tétrabutylammonium, de méthyltri(n-butyl)ammonium, de N-méthyl-N,N,N-trioctylammonium, de triméthylphénylphosphonium, de tétrabutylphosphonium, de méthyltri(n-butyl)phosphonium, de méthyltri(isobutyl)phosphonium, de diisobutyl-n-octylméthylphosphonium. On choisit préférentiellement le fluorure de tétrabutylammonium et le fluorure de tétrabutylphosphonium.

De manière préférée, ledit fluorure ionique d'un cation divalent est tel que ledit cation est un cation alcalino-terreux choisi parmi préférentiellement le magnésium et le calcium ou un cation appartenant au groupe IIB de la classification périodique des éléments, préférentiellement le zinc.

Conformément au procédé de l'invention opéré en phase liquide, il est avantageux de mettre en oeuvre un mélange de fluorures ioniques tels que définis ci-dessus. En particulier, il est avantageux d'utiliser un mélange de fluorures de cations monovalents, et très préférentiellement un mélange d'un fluorure de potassium et d'un fluorure d'onium tel que précédemment défini.

La quantité de fluorure ionique (monovalent et/ou divalent) utilisée par rapport à la quantité de composé de formule (II) est de préférence supérieure à la stoechiométrie. Le rapport du nombre de moles fluorure au nombre d'atomes d'halogène à échanger du composé (II) est avantageusement compris entre 1 et 20, de préférence 4 à 10.

Le procédé selon l'invention opéré en phase liquide est mis en oeuvre en milieu aqueux, en milieu hydro-organique ou en milieu organique.

Le solvant organique présent dans le milieu hydro-organique ou milieu organique anhydre est préférentiellement un solvant aprotique polaire, en particulier dans un solvant sulfoxyde tel que le diméthylsulfoxyde (DMSO), dans un solvant amine N,N disubstitué tel que le diméthylformamide (DMF), dans un solvant nitrile tel que l'acétonitrile ou l'adiponitrile, dans un solvant ester tel que l'acétate d'éthyle, dans un solvant amine tertiaire tel que la triéthylamine, dans un solvant hétérocycle azoté tel que la pyridine, dans un solvant cétone tel que l'acétone ou la butanone, dans un solvant organosoufré tel que le sulfolane.

Le procédé selon l'invention opéré en phase liquide est mis en oeuvre à une température comprise entre 80 et 400°C. Il est conduit sous pression régulée ou sous pression autogène.

II peut être mis en oeuvre en continu ou en discontinu.
Les réactifs mis en oeuvre dans le procédé selon l'invention opéré en phase liquide peuvent être introduits dans n'importe quel ordre selon différentes variantes.

Lorsque le procédé selon l'invention est opéré en milieu aqueux ou hydro-organique, un mode préféré de réalisation consiste à mélanger l'eau, éventuellement additionnée d'un solvant organique, et au moins un fluorure ionique tel que défini plus haut dans la présente description, notamment le fluorure de potassium. Ce mélange est chauffé à la température de réaction voulue, notamment comprise entre 80 et 250°C, de préférence entre 100 et 180°C, puis ledit composé de formule (II) y est introduit. Le mélange réactionnel est avantageusement agité pendant toute la durée durant laquelle le chauffage est maintenu. Le composé de formule (II) est introduit pur, en solution dans l'eau ou dans ledit solvant organique ou dans un mélange eau-soivant. Ledit composé de formule (II) peut être introduit en une seule fois ou progressivement, par fraction. Un autre mode de réalisation préféré du procédé selon l'invention, opéré en milieu aqueux ou hydro-organique, consiste à introduire simultanément au moins fluorure ionique et ledit composé de formule (II) dans l'eau, éventuellement additionnée d'un solvant organique, puis à chauffer ledit mélange réactionnel à la température de réaction voulue. Le chauffage du mélange réactionnel est maintenu pendant une durée variable. De manière préférée, le chauffage du mélange réactionnel est maintenu pendant une durée comprise entre 30 minutes et 48 heures, de manière plus préférée entre 1 et 10 heures et de manière encore plus préférée entre 1 et 5 heures.

Lorsque le procédé selon l'invention est opéré en milieu organique, un mode de réalisation préféré consiste à introduire le composé de formule (II), pur ou présent en solution dans ledit solvant aprotique polaire, sur une suspension d'au moins un fluorure ionique dans ledit solvant, ladite suspension ayant été préalablement chauffée à la température choisie préférentiellement entre 200 et 400°C. Le chauffage du mélange réactionnel est maintenu pendant une durée variant entre 2 et 20 heures, préférentiellement entre 2 et 10 heures.

Le composé de formule (I), obtenu selon le procédé de l'invention opéré en phase liquide, est récupéré, à partir du milieu réactionnel, selon toutes les techniques classiques connues de l'Homme du métier, par exemple par extraction liquide-liquide suivie d'une purification par cristallisation ou distillation.

Selon un mode particulier de réalisation du procédé selon l'invention, l'agent de fluoration choisi parmi l'acide fluorhydrique et un fluorure ionique d'un cation monovalent ou divalent est avantageusement employé en association avec un second agent de fluoration tel que le gaz F₂, le tétrafluorure de soufre SF₄, l'hexafluorure de soufre SF₆ ou le fluorure de thionyle SOF₂. On pourrait encore mettre en oeuvre le procédé de l'invention simplement au moyen de gaz F₂, de tétrafluorure de soufre SF₄, d'hexafluorure de soufre SF₆ et/ou de fluorure de thionyle SOF₂.

Le procédé de l'invention, opéré en phase gazeuse ou phase liquide, est avantageusement conduit dans un appareillage susceptible de résister à la corrosion du milieu réactionnel. Par exemple, on choisit un appareillage formé d'un matériau graphite ou de polymères fluorés (notamment le polytétrafluoroéthylène PTFE, le polyfluorure de vinylidène PVDF et les résines perfluoroalkyles PFA). L'appareillage peut encore être formé d'alliages à base de molybdène, chrome, cobalt, fer, cuivre, manganèse, titane, zirconium, aluminium, carbone et tungstène vendus sous les marques HASTELLOY® ou d'alliages de nickel, chrome, fer, manganèse additivés de cuivre et/ou molybdène commercialisés sous la dénomination INCONEL® et plus particulièrement d'alliages HASTELLOY C 276 ou INCONEL 600, 625 ou 718. On peut choisir également les aciers inoxydables, tels que les aciers austénitiques [Robert H. Perry et al, Perry's Chemical Engineers' Handbook, Sixth Edition (1984), page 23-44]. et plus particulièrement les aciers inoxydables 304, 304 L, 316 ou 316 L.

Conformément au procédé de l'invention, le composé de formule (II) est avantageusement obtenu par halogénation radicalaire, préférentiellement par chloration radicalaire, d'un composé de formule R'_{H}-SO₂X (formule III), où R'_{H} est choisi parmi les groupements R'_{H}1, R'_{H}2 et R'_{H}3 suivants :
R'_{H}1 = -CₙH₂ₙ₊₁ avec n = 1 ;
R'_{H}2 = -CₓH₂ₓ-SO₂X avec x = 1 ;
R'_{H}3 = Φ-C_{c}H_{2c} avec c = 1 ;
X étant un atome d'halogène choisi parmi le chlore et le brome.

Plus particulièrement, la chloration radicalaire de R'_{H}1-SO₂X conduit à l'obtention du composé de formule R'1-SO₂X avec R'1 tel que défini ci-avant. La chloration radicalaire de R'_{H}2-SO₂X conduit à l'obtention du composé de formule R'2-SO₂X avec R'2 tel que défini ci-avant. La chloration radicalaire de R'_{H}3-SO₂X conduit à l'obtention du composé de formule R'3-SO₂X avec R'3 tel que défini ci-avant. X est un atome d'halogène choisi parmi le chlore et le brome.

L'halogénation radicalaire, préférentiellement la chloration radicalaire, est un procédé connu de l'Homme du métier. L'Homme du métier pourra par exemple facilement mettre en oeuvre un procédé d'halogénation radicalaire à partir de l'enseignement décrit dans US 2.674.620. L'halogénation radicalaire, préférentiellement la chloration radicalaire, est mise en oeuvre par photo-halogénation, préférentiellement par photo-chloration.

Le composé de formule (II) est encore avantageusement obtenu par halogénation ionique, plus particulièrement par chloration ionique. L'Homme du métier pourra par exemple facilement mettre en oeuvre un procédé d'halogénation ionique à partir de l'enseignement décrit dans US 2.832.803.

De manière préférée, le composé de formule (III) est un composé de formule R'_{H}1-SO₂X avec R'_{H}1 = -CₙH₂ₙ₊₁, n = 1 et X = Cl. Ainsi, on procède à la préparation de CCl₃-SO₂Cl par chloration radicalaire du chlorure de mésyle de formule CH₃-SO₂Cl.

Le composé fluoré de formule (I) préparé selon le procédé de l'invention est avantageusement utilisé en tant que composé réactif pour la synthèse d'un composé sulfonimide (R-SO₂)₂NH et de ses sels (R-SO₂)₂NMe (Me représentant un métal alcalin) ou d'un composé fluoré ayant une fonction acide sulfonique -SO₂OH et présentant une formule R-SO₂OH, R ayant la définition précisée plus haut dans la présente description, à savoir choisi parmi les groupements R1, R2 et R3.

L'invention a également pour objet un procédé de préparation d'un composé choisi dans le groupe constitué par un composé sulfonimide (R-SO₂)₂NH, ses sels (R-SO₂)₂NMe (Me représentant un métal alcalin) et un composé fluoré ayant une fonction acide sulfonique -SO₂OH et présentant une formule R-SO₂OH, R ayant la définition précisée plus haut dans la présente description, ledit procédé comprenant :
- une étape de préparation d'un composé R-SO₂F de formule (I) selon le procédé décrit ci-avant,
- une étape dans laquelle ledit composé fluoré de formule (I) est utilisé en tant que composé réactif pour la synthèse d'un composé sulfonimide (R-SO₂)₂NH et de ses sels (R-SO₂)₂NMe (Me représentant un métal alcalin) ou d'un composé fluoré ayant une fonction acide sulfonique -SO₂OH et présentant une formule R- SO₂OH, R ayant la définition précisée plus haut dans la présente description.

La présente invention a donc également pour objet un procédé de préparation d'un sel d'un composé sulfonimide de formule (R-SO₂)₂NMe à partir d'un composé fluoré de formule (I) comprenant :
a) la préparation d'un composé R-SO₂F de formule (I) selon le procédé décrit ci-avant ;
b) une étape d'ammonolyse de R-SO₂F en (R-SO₂)₂NH, NR"₃ ;
c) une étape d'acidification de (R-SO₂)₂NH, NR"₃ en (R-SO₂)₂NH ;
d) une étape de neutralisation, par une base de métal alcalin, de (R-SO₂)₂NH en (R-SO₂)₂NMe ; et
e) éventuellement une étape de séchage de (R-SO₂)₂NMe,
dans lequel R est choisi parmi les radicaux R1, R2 et R3 définis ci-avant, R" représente un groupe alkyle, linéaire ou branché, ayant de 1 à 20 atomes de carbone, Me représente un métal alcalin. De manière préférée, Me est le lithium.
Les étapes b), c), d) et e) sont connues de l'Homme du métier. En particulier, l'étape d'ammonolyse est décrite dans le brevet US 5.723.664, Les étapes d'acidification, de neutralisation et de séchage sont des étapes classiques pouvant être effectuées dans les conditions connues de l'Homme du métier.

De manière préférée, le composé fluoré de formule (I) est le fluorure de trifluorométhane sulfonyle (CF₃SO₂F) de manière à pouvoir l'utiliser dans la synthèse du bis-(trifluorométhanesulfonyl)imide de formule (CF₃SO₂)₂NH et du bis-(trifluorométhanesulfonyl)imidure de lithium de formule (CF₃SO₂)₂NLi (LiTFSI).

Les composés sulfonimides et leurs sels préparés selon les procédés décrits ci-dessus peuvent avantageusement être utilisés comme sels d'électrolyte, comme précurseurs d'agent antistatique ou encore comme précurseurs de tensio-actif. En particulier, lesdits composés peuvent avantageusement être employés comme électrolytes pour la fabrication de batteries, dans le domaine de l'électrochromisme, de l'électronique et de l'électrochimie. Ils sont avantageusement employés comme agents antistatiques pour la fabrication d'adhésifs sensibles à la pression (PSA : pressure sensitive adhesives). En tant qu'agent antistatique, ils peuvent encore être employés comme composants de lubrifiants. Ils sont utilisés dans les matériaux optiques tels que les appareils électroluminescents et entrent dans la composition de panneaux photovoltaïques. Ces utilisations sont également des objets de l'invention. En particulier, l'invention a pour objet un procédé de fabrication d'un dispositif électrochimique, de préférence une batterie, ledit procédé comprenant une étape de préparation d'un composé sulfonimide ou de ses sels selon le procédé décrit ci-avant, et une étape de fabrication du dispositif électrochimique dans lequel le composé sulfonimide ou ses sels est employé comme électrolyte.

Le composé de formule (I) préparé selon le procédé de l'invention est encore avantageusement utilisé pour la préparation, par hydrolyse, d'un composé fluoré de formule R-SO₂-OH où R est choisi parmi les radicaux R1, R2 et R3 définis ci-avant. A cet effet, on met par exemple le flux gazeux comprenant le composé fluoré de formule (I) issu du procédé de l'invention, opéré en phase gazeuse, en présence d'une solution aqueuse alcaline puis on procède à une étape d'acidification pour libérer le composé R-SO₂-OH, par exemple en utilisant une solution d'un acide minéral fort, tel que l'acide sulfurique ou l'acide chlorhydrique.

De manière préférée, le composé fluoré de formule (I) est le fluorure de trifluorométhane sulfonyle (CF₃SO₂F) de manière à pouvoir l'utiliser dans la synthèse de l'acide trifluorométhanesulfonique (appelé acide triflique) de formule CF₃SO₂OF.

Le composé R-SO₂-OH ainsi obtenu est avantageusement transformé en anhydride de formule (R-SO₂)₂O. La réaction d'anhydrisation est connue de l'Homme du métier et est particulièrement décrite dans le brevet US 8.222.450. De manière préférée, le composé fluoré de formule (I) est le fluorure de trifluorométhane sulfonyle (CF₃SO₂F) de manière à ce que l'anhydrisation de l'acide triflique conduise à la production de l'anhydride trifluorométhanesulfonique de formule (CF₃SO₂)₂O.

La présente invention va maintenant être décrite à l'aide d'exemples non limitatifs.

### Exemples 1 à 7 : Préparation de TFSF par une réaction de fluoration par HF en phase gazeuse

Dans un réacteur en Hastelloy C276 constitué d'un tube de 60 cm de longueur et de diamètre extérieur de 2,5 cm, rempli d'un catalyseur à base d'oxyde de chrome (∼150g) préalablement séché jusqu'à poids constant et fluoré, on introduit du chlorure de trichlorométhanesulfonyle (TCSC), pur ou dissous dans un solvant, à un débit de 0,05 mol/h de TCSC et de l'HF anhydre à un débit de 10 g/h. Le solvant est le trifluorométhylbenzène (TFMB), le trifluorométhoxybenzène (TFMxB) ou le toluène.
La température est, selon l'essai, fixée de 200°C à 300°C isotherme. Dans ces conditions, le temps de séjour tₛ varie environ entre 10 et 25 s.
Après réaction, le flux sortant composé de fluorure de trifluorométhane sulfonyle, d'HF et d'HCl, est hydrolysé dans des barboteurs à potasse montés en série, et les différents acides sont dosés sous forme de sels de potassium par chromatographie ionique. Le dosage de TFSF (CF₃SO₂F) est réalisé sous forme de triflate de potassium (CF₃SO₂K).

Les résultats obtenus figurent dans le tableau (I).
Le taux de conversion TT correspond au rapport entre le nombre de moles de substrat TCSC transformées et le nombre de moles de substrat TCSC engagées.
Le rendement RR correspond au rapport entre le nombre de moles de produit fluorure de trifluorométhane sulfonyle TFSF formées et le nombre de moles de substrat TCSC engagées.
Le rendement RT correspond au rapport entre le nombre de moles de produit TFSF formées et le nombre de moles de substrat TCSC transformées.

**Tableau (I)**

| Réf. ex | Solvant | T°C | tₛ (s) | HF/TCSC (mol) | TT % | RR % | RT % |
|---|---|---|---|---|---|---|---|
| 1 | - | 250 | 22 | 10,8 | 82 | 75 | 91 |
| 2 | - | 250 | 11,5 | 21 | 50 | 47 | 94 |
| 3 | TFMB | 250 | 18 | 11 | 74 | 70 | 95 |
| 4 | TFMxB | 250 | 12,6 | 10,7 | 45 | 42 | 93 |
| 5 | toluène | 250 | 12,3 | 10,9 | 46 | 41 | 89 |
| 6 | - | 200 | 25 | 10,2 | 42 | 41 | 97 |
| 7 | - | 300 | 20,3 | 10,6 | 98 | 80 | 82 |

### Exemple 8 : Préparation de TFSF par une réaction de fluoration par HF en phase liquide

Dans un autoclave en Hastelloy C276 de 280ml de capacité, on charge :

| | |
|---|---|
| - TCSC : | 110g (0,5 mol) |
| - HF : | 40g (2 mol, soit ∼4équi.) |
| - SbCl₅ : | 5g (0,02 mol, soit ∼1% mol par rapport à HF) |

L'autoclave est porté à 120°C pendant 3h sous pression autogène, puis refroidi à 20°C et dégazé dans des barboteurs à potasse montés en série ; le milieu réactionnel résiduel est soutiré et abattu dans de la potasse aqueuse.

Les phases aqueuses potassiques sont rassemblées et analysées par ¹⁹F RMN ; le fluorure de trifluorométhanesulfonyle (TFSF), dosé sous forme de triflate de potassium (TAK de formule CF₃SO₃K), est obtenu avec un rendement de 23%.

### Exemple 9 : Préparation de TFSF par une réaction de fluoration par KF

### • Exemple 9.1 : Dans un solvant aprotique polaire

Dans un autoclave en acier inoxydable de nuance 316L et de capacité 150ml, on introduit :

| | |
|---|---|
| - KF : | 29g |
| - TCSC : | 22g |
| - Adiponitrile : | 60ml |

L'autoclave est fermé et porté à 230°C sous pression autogène pendant 4h, puis refroidi à 20°C et dégazé dans des barboteurs à potasse montés en série ; le milieu réactionnel résiduel est soutiré et abattu dans de la potasse aqueuse.

Les phases aqueuses potassiques sont rassemblées et analysées par ¹⁹F RMN ; le fluorure de trifluorométhanesulfonyle, dosé sous forme de triflate de potassium (TAK), est obtenu avec un rendement de 47%.

### • Exemple 9.2 : Dans l'eau

Dans un réacteur en verre parfaitement agité de 100ml de capacité sont introduits :

| | |
|---|---|
| - KF : | 32,6g |
| - TCSC : | 12g |
| - eau : | 20ml |

Le milieu est porté à ébullition sous agitation pendant 1h, puis refroidi et ramené à pH neutre par addition de potasse aqueuse.

L'analyse du milieu par ¹⁹F RMN montre que le triflate de potassium (TAK) a été formé avec un rendement de 63%.

### Exemple 10 : préparation de DFSF (CHF₂SO₂F) par une réaction de fluoration par HF en phase gazeuse

La réaction est conduite dans les mêmes conditions que l'exemple 1, avec les charges et conditions suivantes :
- DCSC (CHCl₂SO₂Cl) : 0,05 mol/h
- HF : 10 g/h (rapport HF/DCSC : 10)

La température est fixée de 250°C isotherme et le temps de séjour tₛ est de 22 s.

Le taux de conversion TT du DCSC est de 65% et le rendement RR en DFSF est de 42%.

### Exemple 11 : préparation du difluorure de difluorométhanedisulfonvyle (DF₂DS : (CF₂(SO₂F)₂) par une réaction de fluoration par HF en phase liquide

La réaction est conduite dans les mêmes conditions que l'exemple 8, avec les charges et conditions suivantes :
- (CCl₂(SO₂Cl)₂) : 100 g (0,35 mol)
- HF : 40 g (2 mol, soit ∼6 éq.)

Après 3h de réaction à 120°C, le milieu réactionnel est traité selon l'exemple 8. Le DF₂DS est obtenu avec un rendement de 28%.

### Exemple 12 : préparation du fluorure de α,α-difluorobenzylsulfonyle (DFBSF : (C₆H₅CF₂SO₂F) par une réaction de fluoration par HF en phase liquide

La réaction est conduite dans les mêmes conditions que l'exemple 8, avec les charges et conditions suivantes :
- C₆H₅CCl₂SO₂Cl : 100 g (0,4 mol)
- HF : 40 g (2 mol, soit ∼5 éq.)

Après 4h de réaction à 150°C, le milieu réactionnel est traité selon l'exemple 8. Le DFBSF est obtenu avec un rendement de 19%.

## Revendications

1. Procédé non-électrochimique de préparation d'un composé fluoré de formule (I) comportant au moins une fonction -SO₂F **caractérisé en ce que** le composé de formule (I) est préparé par réaction d'un composé de formule (II) avec au moins un agent de fluoration choisi parmi l'acide fluorhydrique et un fluorure ionique d'un cation monovalent ou divalent :
R-SO₂F (I)
Où R est choisi parmi les groupements R1, R2 et R3 suivants :
R1 = -CₙHₐF_{b} avec n = 1, a+b = 3, b ≥ 1 ;
R2 = -CₓH_{y}F_{z}-SO₂F avec x = 1, y+z = 2 et z ≥ 1 ;
R3 = Φ-C_{c}HₕF_{f} avec c = 1; h+f = 2 et f ≥ 1 ; Φ désignant un groupe phényle
R'-SO₂X (II)
Où R' est choisi parmi les groupements R'1, R'2 et R'3 suivants :
R'1 = -CₙHₐX_{b} avec n = 1, a+b = 3, b ≥ 1 ;
R'2 = -CₓH_{y}X_{z}-SO₂X avec x = 1, y+z = 2 et z ≥ 1 ;
R'3 = Φ-C_{c}HₕX_{f} avec c = 1 ; h+f = 2 et f ≥ 1 ; Φ désignant un groupe phényle,
X étant un atome d'halogène choisi parmi le chlore et le brome.

2. Procédé de préparation selon la revendication 1 tel que les radicaux R1 et R'1 sont perhalogénés de sorte que b = 3 et a = 0.

3. Procédé de préparation selon la revendication 1 tel que le radical R du composé (I) est le radical R1 dans lequel a = 0 et b = 3, ou a = 1, b = 2 ou encore a = 2 et b = 1.

4. Procédé de préparation selon l'une des revendications 1 à 3 tel qu'il est opéré en phase gazeuse et que l'agent de fluoration est l'acide fluorhydrique.

5. Procédé de préparation selon la revendication 4 tel qu'il met en oeuvre au moins un catalyseur de fluoration comprenant, ou consistant en, du chrome, du zinc, du nickel, un mélange de chrome et de zinc ou un mélange de chrome et de nickel.

6. Procédé de préparation selon la revendication 4 ou la revendication 5 tel que le rapport entre le nombre de moles d'acide fluorhydrique et le nombre de moles de composé halogéné de formule (II) varie entre 1 et 30.

7. Procédé de préparation selon l'une des revendications 1 à 3 tel qu'il est opéré en phase liquide en présence d'acide fluorhydrique au moyen d'un catalyseur de fluoration à base d'antimoine.

8. Procédé de préparation selon l'une des revendications 1 à 3 tel qu'il est opéré en phase liquide en présence d'un fluorure ionique d'un cation monovalent choisi parmi un fluorure d'un cation alcalin et un fluorure d'un cation onium.

9. Procédé de préparation selon la revendication 8 tel que ledit cation alcalin est le potassium.

10. Procédé de préparation selon la revendication 8 tel que ledit fluorure d'un cation onium est choisi parmi un fluorure d'ammonium dans lequel le cation répond à la formule N(R₄R₅R₆R₇)⁺ et un fluorure de phosphonium dans lequel le cation répond à la formule P(R₄R₅R₆R₇)⁺, R₄, R₅, R₆ et R₇, identiques ou différents, sont choisis parmi un groupe alkyle, linéaire ou ramifié, ayant 1 à 12 atomes de carbone et un groupe benzyle.

11. Procédé de préparation selon l'une des revendications 7 à 10 tel qu'il est mis en oeuvre en milieu aqueux, en milieu hydro-organique ou en milieu organique.

12. Procédé de préparation selon l'une des revendications 1 à 11 tel que le composé de formule (II) est obtenu par halogénation radicalaire d'un composé de formule R'_{H}-SO₂X (formule III), où R'_{H} est choisi parmi les groupements R'_{H}1, R'_{H}2 et R'_{H}3 suivants :
R'_{H}1 = -CₙH₂ₙ₊₁ avec n = 1,
R'_{H}2 = -CₓH₂ₓ-SO₂X avec x = 1 ;
R'_{H}3 = Φ-C_{c}H_{2c} avec c = 1 ;
X étant un atome d'halogène choisi parmi le chlore et le brome.

13. Procédé de préparation d'un composé choisi dans le groupe constitué par un composé sulfonimide (R-SO₂)₂NH, ses sels (R-SO₂)₂NMe et un composé fluoré ayant une fonction acide sulfonique -SO₂OH et présentant une formule R-SO₂OH, R ayant la définition de la revendication 1, ledit procédé comprenant :
- une étape de préparation d'un composé R-SO₂F de formule (I) selon l'une quelconque des revendications 1 à 12,
- une étape dans laquelle ledit composé fluoré de formule (I) est utilisé en tant que composé réactif pour la synthèse d'un composé sulfonimide (R-SO₂)₂NH et de ses sels (R-SO₂)₂NMe ou d'un composé fluoré ayant une fonction acide sulfonique - SO₂OH et présentant une formule R- SO₂OH, R ayant la définition de la revendication 1.

14. Procédé selon la revendication 13 pour la synthèse du bis-(trifluorométhanesulfonyl)imide de formule (CF₃SO₂)₂NH et du bis-(trifluorométhanesulfonyl)imidure de lithium de formule (CF₃SO₂)₂NLi (LiTFSI).

15. Procédé selon la revendication 13 pour la synthèse de l'acide trifluorométhanesulfonique de formule CF₃SO₂OH.

## Patentansprüche

1. Nicht-elektrochemisches Verfahren zur Herstellung einer fluorierten Verbindung mit der Formel (I), die mindestens eine Gruppe -SO₂F aufweist, **dadurch gekennzeichnet, dass** die Verbindung mit der Formel (I) durch eine Reaktion einer Verbindung mit der Formel (II) mit mindestens einem Fluorierungsmittel hergestellt wird, das ausgewählt wird aus Flusssäure und einem ionischen Fluorid eines einwertigen oder zweiwertigen Kations:
R-SO₂F (I),
wobei R aus den folgenden Gruppen R1, R2 und R3 ausgewählt wird:
R1 = -CₙHₐF_{b} mit n = 1, a+b = 3, b ≥ 1;
R2 = -CₓH_{y}F_{z}-SO₂F mit x = 1, y+z = 2 und z ≥ 1;
R3 = Φ-C_{c}HₕF_{f} mit c = 1 ; h+f = 2 und f ≥ 1; wobei Φ eine Phenylgruppe bezeichnet,
R'-SO₂X (II),
wobei R' aus den folgenden Gruppen R'1, R'2 und R'3 ausgewählt wird:
R'1 = -CₙHₐX_{b} mit n = 1, a+b = 3, b ≥ 1;
R'2 = -CₓH_{y}X_{z}-SO₂X mit x = 1, y+z = 2 und z ≥ 1;
R'3 = Φ-C_{c}HₕX_{f} mit c = 1; h+f = 2 und f ≥ 1; wobei Φ eine Phenylgruppe bezeichnet,
wobei X ein Halogenatom ist, das aus Chlor und Brom ausgewählt wird.

2. Verfahren zur Herstellung nach Anspruch 1, wobei die Reste R1 und R'1 perhalogeniert werden, sodass b = 3 und a = 0.

3. Verfahren zur Herstellung nach Anspruch 1, wobei der Rest R der Verbindung (I) der Rest R1 ist, bei dem a = 0 und b = 3 oder a = 1, b = 2 oder auch a = 2 und b = 1.

4. Verfahren zur Herstellung nach einem der Ansprüche 1 bis 3, wobei es in der Gasphase durchgeführt wird und das Fluorierungsmittel Flusssäure ist.

5. Verfahren nach Anspruch 4, wobei darin mindestens ein Fluorierungskatalysator eingesetzt wird, der Chrom, Zink, Nickel, eine Mischung aus Chrom und Zink oder eine Mischung aus Chrom und Nickel umfasst oder daraus besteht.

6. Verfahren zur Herstellung nach Anspruch 4 oder nach Anspruch 5, wobei das Verhältnis zwischen der Stoffmenge an Flusssäure und der Stoffmenge an halogenierter Verbindung mit der Formel (II) zwischen 1 und 30 liegt.

7. Verfahren zur Herstellung nach einem der Ansprüche 1 bis 3, wobei es in der Flüssigphase in Gegenwart von Flusssäure mit einem Fluorierungskatalysator auf Basis von Antimon durchgeführt wird.

8. Verfahren zur Herstellung nach einem der Ansprüche 1 bis 3, wobei es in der Flüssigphase in Gegenwart eines ionischen Fluorids eines einwertigen Kations durchgeführt wird, das aus einem Fluorid eines Alkalimetallkations und einem Fluorid eines Onium-Kations ausgewählt wird.

9. Verfahren zur Herstellung nach Anspruch 8, wobei das Alkalimetallkation Kalium ist.

10. Verfahren zur Herstellung nach Anspruch 8, wobei das Fluorid eines Onium-Kations ausgewählt wird aus einem Ammoniumfluorid, in dem das Kation der Formel N(R₄R₅R₆R₇)⁺ entspricht, und einem Phosphoniumfluorid, in dem das Kation der Formel P(R₄R₅R₆R₇)⁺ entspricht, wobei R₄, R₅, R₆ und R₇, identisch oder unterschiedlich, aus einer unverzweigten oder verzweigten Alkylgruppe mit 1 bis 12 Kohlenstoffatomen und einer Benzylgruppe ausgewählt werden.

11. Verfahren zur Herstellung nach einem der Ansprüche 7 bis 10, wobei es im wässrigen Medium, im wässrig-organischen Medium oder im organischen Medium durchgeführt wird.

12. Verfahren zur Herstellung nach einem der Ansprüche 1 bis 11, wobei die Verbindung mit der Formel (II) durch radikalische Halogenierung einer Verbindung mit der Formel R'_{H}-SO₂X (Formel III) erhalten wird, wobei R'_{H} aus folgenden Gruppen R'_{H}1, R'_{H}2 und R'_{H}3 ausgewählt wird:
R'_{H}1 = -CₙH₂ₙ₊₁ mit n = 1,
R'_{H}2 = -CₓH₂ₓ-SO₂X mit x = 1;
R'_{H}3 = Φ-C_{c}H_{2c} mit c = 1;
wobei X ein Halogenatom ist, das aus Chlor und Brom ausgewählt wird.

13. Verfahren zur Herstellung einer Verbindung, die aus der Gruppe ausgewählt wird, die von einer Sulfonimidverbindung (R-SO₂)₂NH, ihren Salzen (R-SO₂)₂NMe und einer fluorierten Verbindung mit einer Sulfonsäuregruppe -SO₂OH und einer Formel R-SO₂OH gebildet wird, wobei R der Definition von Anspruch 1 entspricht, wobei das Verfahren umfasst:
- einen Schritt zur Herstellung einer Verbindung R-SO₂F mit der Formel (I) nach einem der Ansprüche 1 bis 12,
- einen Schritt, in dem die fluorierte Verbindung mit der Formel (I) als reaktionsfähige Verbindung für die Synthese einer Sulfonimidverbindung (R-SO₂)₂NH und ihrer Salze (R-SO₂)₂NMe oder einer fluorierten Verbindung mit einer Sulfonsäuregruppe -SO₂OH und einer Formel R-SO₂OH verwendet wird, wobei R der Definition von Anspruch 1 entspricht.

14. Verfahren nach Anspruch 13 zur Synthese von bis-(Trifluormethansulfonyl)imid mit der Formel (CF₃SO₂)₂NH und von Lithium-bis-(trifluormethansulfonyl) imid mit der Formel (CF₃SO₂)₂NLi (LiTFSI) .

15. Verfahren nach Anspruch 13 zur Synthese von Trifluormethansulfonsäure mit der Formel CF₃SO₂OH.

## Claims

1. Non-electrochemical process for the preparation of a fluorinated compound of formula (I) comprising at least one -SO₂F functional group, **characterized in that** the compound of formula (I) is prepared by reaction of a compound of formula (II) with at least one fluorinating agent chosen from hydrofluoric acid and an ionic fluoride of a monovalent or divalent cation:
R-SO₂F (I)
where R is chosen from the following groups R1, R2 and R3:
R1 = -CₙHₐF_{b} with n = 1, a+b = 3, b ≥ 1;
R2 = -CₓH_{y}F_{z}-SO₂F with x = 1, y+z = 2 and z ≥ 1;
R3 = Φ-C_{c}HₕF_{f} with c = 1, h+f = 2 and f ≥ 1, Φ denoting a phenyl group,
R'-SO₂X (II)
where R' is chosen from the following groups R'1, R'2 and R'3:
R'1 = -CₙHₐX_{b} with n = 1, a+b = 3, b ≥ 1;
R'2 = -CₓH_{y}X_{z}-SO₂F with x = 1, y+z = 2 and z ≥ 1;
R'3 = Φ-C_{c}HₕX_{f} with c = 1, h+f = 2 and f ≥ 1, Φ denoting a phenyl group,
X being a halogen atom chosen from chlorine and bromine.

2. Preparation process according to Claim 1, such that the R1 and R'1 radicals are perhalogenated so that b = 3 and a = 0.

3. Preparation process according to Claim 1, such that the R radical of the compound (I) is the R1 radical in which a = 0 and b = 3, or a = 1 and b = 2, or else a = 2 and b = 1.

4. Preparation process according to one of Claims 1 to 3, such that it is carried out in the gas phase and that the fluorinating agent is hydrofluoric acid.

5. Preparation process according to Claim 4, such that it employs at least one fluorination catalyst comprising, or consisting of, chromium, zinc, nickel, a mixture of chromium and zinc or a mixture of chromium and nickel.

6. Preparation process according to Claim 4 or Claim 5, such that the ratio of the number of moles of hydrofluoric acid to the number of moles of halogenated compound of formula (II) varies between 1 and 30.

7. Preparation process according to one of Claims 1 to 3 such that it is carried out in the liquid phase in the presence of hydrofluoric acid by means of an antimony-based fluorination catalyst.

8. Preparation process according to one of Claims 1 to 3, such that it is carried out in the liquid phase in the presence of an ionic fluoride of a monovalent cation chosen from a fluoride of an alkali metal cation and a fluoride of an onium cation.

9. Preparation process according to Claim 8, such that the said alkali metal cation is potassium.

10. Preparation process according to Claim 8, such that the said fluoride of an onium cation is chosen from an ammonium fluoride in which the cation corresponds to the formula N(R₄R₅R₆R₇)⁺ and a phosphonium fluoride in which the cation corresponds to the formula P(R₄R₅R₆R₇)⁺; R₄, R₅, R₆ and R₇, which are identical or different, are chosen from a linear or branched alkyl group having from 1 to 12 carbon atoms and a benzyl group.

11. Preparation process according to one of Claims 7 to 10, such that it is carried out in an aqueous medium, in an aqueous/organic medium or in an organic medium.

12. Preparation process according to one of Claims 1 to 11, such that the compound of formula (II) is obtained by radical halogenation of a compound of formula R'_{H}-SO₂X (formula III), where R'_{H} is chosen from the following groups R'_{H}1, R'_{H}2 and R'_{H}3:
R'_{H}1 = -CₙH₂ₙ₊₁ with n = 1;
R'_{H}2 = -CₓH₂ₓ-SO₂X with x = 1;
R'_{H}3 = Φ-C_{c}H_{2c} with c = 1;
X being a halogen atom chosen from chlorine and bromine.

13. Process for the preparation of a compound chosen from the group consisting of a sulfonimide compound (R-SO₂)₂NH, its (R-SO₂)₂NMe salts and a fluorinated compound having a sulfonic acid -SO₂OH functional group and exhibiting a formula R-SO₂OH, R having the definition of Claim 1, the said process comprising:
- a stage of preparation of an R-SO₂F compound of formula (I) according to any one of Claims 1 to 12,
- a stage in which the said fluorinated compound of formula (I) is used as reactive compound for the synthesis of a sulfonimide compound (R-SO₂)₂NH and of its (R-SO₂)₂NMe salts or of a fluorinated compound having a sulfonic acid -SO₂OH functional group and exhibiting a formula R-SO₂OH, R having the definition of Claim 1.

14. Process according to Claim 13 for the synthesis of bis(trifluoromethanesulfonyl)imide of formula (CF₃SO₂)₂NH and of lithium bis(trifluoromethanesulfonyl)imide of formula (CF₃SO₂)₂NLi (LiTFSI).

15. Process according to Claim 13 for the synthesis of trifluoromethanesulfonic acid of formula CF₃SO₂OH.
